# EUROPEAN PATENT APPLICATION

(11) **EP 1 059 531 A1**
(43) Date of publication of application: **13.12.2000**
(21) Application number: 99111176.6
(22) Date of filing: 09.06.1999
(51) Int. Cl.: G01N 33/534, G01N 33/60, C07K 1/26, C07K 1/28

(54) **Labelling of peptides and proteins**

(71) Applicant: Nordheim, Alfred, Prof. Dr., 72135 Dettenhausen (DE); Cahill, Michael, Dr., 72116 Moessingen (DE)
(72) Inventor: Nordheim, Alfred, Prof. Dr., 72135 Dettenhausen (DE); Cahill, Michael, Dr., 72116 Moessingen (DE)
(74) Representative: Patentanwälte Ruff, Beier und Partner

(57) **Abstract**

This invention relates to methods for labelling peptides and/or proteins in preparative and analytical electrophoresis, especially as sample preparation for isoelectric focussing, wherein side chains of specific amino acid residues of the peptides and/or proteins are derivatized and labelled. The purpose of the invention is to control the isoelectric point of the peptides and/or proteins to permit isoelectric focussing of the samples with reliable results. In one embodiment of the invention cysteine residues of the peptides and/or proteins are alkylated, such that the modified amino acid side chain can be labelled, e.g. iodinated, along with tyrosine during standard conditions for tyrosine labelling. The invention permits unincorporated label to be electrophoretically removed from the sample during subsequent electrophoresis, thus dramatically improving the ratio of specific protein-bound signal to non-specific background signal upon isotopic detection.

## Description

This invention relates to methods for labelling peptides and/or proteins in preparative and analytical electrophoresis, especially for isoelectric focussing or other separation methods, which rely on the isoelectric point or net charge of a peptide or protein.

The use of electrophoresis for preparative or analytical purposes is an established technique and several types of electrophoretic apparatus exist for that purposes. These apparatus and their accompanying principles can be divided into three categogies for the purposes required here (1, 12).
a) zone electrophoresis
b) isotachophoresis
c) isoelectric focussing

Zone electrophoresis includes the glycine and tricine buffered SDS-PAGE protein gel systems which are commonly used in the study of proteins known as proteomics.

Isotachophoresis utilises hydrophilic matrices, and typically exhibits high resolution, but low loading capacities. In combination with Free Flow electrophoresis the method can be used for micropreparative purposes (11).

Isoelectric focussing has been performed in either liquid density gradients, in gel gradients (8), or in multicompartment apparatus with isoelectric immobiline based separating gel medium (9). In particular, isoelectric focussing is a key technology in the field of proteomics.

In the field of protein biochemistry, proteomics has recently been of considerable interest. Proteomics is the study of all proteins expressed by a genome of the cells under study. The acronym "PROTEOME" means PROTEins expressed by a genOME (10). Proteomics may eventually reveal the identity and regulation of most proteins expressed within a particular cell-type (13). Current proteomics techniques involve the use of two dimensional polyacrylamide gel electrophoresis (2D-PAGE), whereby proteins are initially separated by differences in isoelectric point by isoelectric focussing in the first dimension. These proteins are then subsequently separated by molecular weight in an SDS gel in the second dimension (8).

With the exception of cases where limited material is available, such as where primary clinical samples are analysed, in most instances proteomics studies are not currently limited by sensitivity, but instead by 2D gel separation connected issues and problems of detection over the broad range of protein abundances. For instance similar 2D patterns are observed when visualising 2 mg of whole cell protein with Coomassie blue or 50 ng protein with a phosphorimager after metabolic labelling to high specific activity using ³⁵S-Methionine. However there is no advantage in visualising 2 mg of the ³⁵S-Methionine-labelled protein with a phosphorimager since discrimination between individual proteins is lost. Thus conventionally, protein loading amounts are determined according to the amount which approaches the resolution limit of the gel at the sensitivity of the particular visualisation system employed. This vividly demonstrates that the limit of resolution of conventional 2D gels is generally not the sensitivity of detection, but instead is often determined by the separating capacity of the gel system.

E.g. in cases where starting material is limited, considerable advantage can be obtained by incorporating different radioactive isotopes of the same element into the proteins of interest from different samples. Further to the advantages of sensitivity, this permits proteins from separate samples to be co-electrophoresed in one gel and quantified totally independently of each other by Multiple Photon Detection (MPD) (3-5), greatly reducing the labor involved and increasing throughput.

By use of MPD a detection limit of 10⁻²¹ mole, i.e. 600 atoms, has been achieved for proteins. MPD is a technique for the detection and measurement of certain radioisotopes at activity levels which are much below the naturally occurring background. This is made possible by the nearly perfect rejection of background events which is achieved through sophisticated signal processing and analysis. Certain isotopes emit both X-rays as electrons move to lower orbitals, and gamma rays as neutrons decompose upon radioactive decay. The high degree of sensitivity of MPD is obtained by considering only signals with coincident X- and gamma-rays, and where the energy profiles of the measured photons correspond to those expected for the isotope in question. The isotopes compatible with MPD come from two broad families of radioisotopes which together include over 100 members appropriate for use as tags. In particular, they include the isotope iodine-125 (¹²⁵I), one of the most commonly used isotopes in biomedical diagnostics.
Additionally, other iodine isotopes can also be visualised by MPD or phosphorimager analysis. By carefully comparing each detected event with the highly specific decay signature of the used radioisotope, MPD is able to reduce the number of background events to less than one per week. MPD permits multi-labelling (up to 16 co-resident labels), good spatial resolution (about 0.3 mm) and very high dynamic range (linearity over nine orders of magnitude in label down to 10⁻²¹ mole). Two significant advantages which are derived from this capability are that substances labelled with appropriate isotopes can be reliably detected at four orders of magnitude lower concentrations, and the amount of isotope which must be used is reduced 1,000-fold to a level much below the naturally occurring background radiation.

As mentioned, MPD enables "multi colour" visualisation of different isotopes, which means that these isotopes can be quantified independently of one another in the absence of cross-talk between signals. Significantly, due to the multicolour ability of MPD, proteins from different samples can be electrophoretically separated on the same gel and quantified totally independently. As a clinical example, healthy control cells can be labelled with ¹³¹I, cancer cells with ¹²⁵I, and metastatic cells with ¹²⁶I. This enables a direct differential display of proteins, and makes redundant much of the graphic analysis which accompanies current proteomic studies. Thus two main advantages of the use of MPD are in the reduction of gel-to-gel variability because samples can be co-electrophoretically separated in the same gel, and in the increased throughput enabled at the currently limiting step of 2D-PAGE. It is to be stressed here that these two factors are currently recognized as bottlenecks in proteomic analysis, and are independent of the improved signal to noise attributes of MPD.

Thus the ability to iodinate proteins provides a much more sensitive method of protein analysis, and permits the generation of reliable differential display data, especially by the use of MPD. Conventionally, proteins can be iodinated by a variety of methods, notably on the amino acids tyrosine and histidine. Conventional methods include Chloramine-T, or IODO-Beads® from Pierce, USA (N-Chloro-benzenesulfonamide). Commercial reagents also exist for amino acid derivatisation which permit iodine to be incorporated at other positions, however these typically alter the pKₐ (negative logarithm of the dissociation constant of the acidic group) of the amino acid in question, and thus greatly alter the isoelectric point of the proteins under examination, and are therefore generally unsuitable for pre-isoelectric focussing labelling of proteins with iodine. The ability to label proteins with iodine prior to isoelectric focussing would be of great importance, because unincorporated iodine could be maintained in the cationic form (I⁻) and could be induced to exit the experimental system by electrophoresis or dialysis. Thus the specific signal to background ratio would be greatly enhanced, and the sensitivity advantages of MPD in particular can be established.

Therefore the invention has the object to provide an amended method for labelling peptides and/or proteins, especially with iodine, in order to permit isoelectric focussing taking advantage of the preferable features of e.g. iodination. This object is solved by a method according to claim 1. Special embodiments of the inventive method are claimed in the following claims 2 to 16. The wording of all claims is hereby made to the content of the specification by reference.

The main principle of the present invention is to control the IEF of peptides and/or proteins in course of labelling the sample in respect to separation methods which rely on the isoelectric point or net charge of the analyte. This is achieved by derivatisation and incorporation of a label into the derivatized molecule or into the derivatizing reagent prior to derivatisation.

Thus, there are two main embodiments of the inventive method. The first embodiment is characterized in that the isoelectric points of the analytes are essentially the same before and after derivatisation. This permits isoelectric analysis of the sample with reliable results. In the second main embodiment of the invention the inventive method is used with the intention of altering (in a defined manner) the isoelectric point of one or more analyte molecules, such as proteins, to facilitate a more efficient analysis, separation, exclusion, or inclusion of said molecules in any particular experimentally obtained fraction.

The inventive method preferably enables the iodination of sulfhydryl group-containing analyte molecules, such as cysteine-containing peptides or proteins, which makes it possible to perform an isoelectric focussing step without having altered the isoelectric points of the analytes. An important step was to recognise that the pKₐ of cysteine (pH 9.1-9.5) (2) is very similar to that of tyrosine (pH 9.7) (2). Thus, alkylation of cysteine with a tyrosine-like molecule retains intact the approximate isoelectric point of the original protein, and permits labelling of both cysteine and tyrosine with iodine using chemistry which traditionally labels only tyrosine with iodine. Importantly, this process permits the electrophoretic removal of iodine which is not covalently attached to proteins. This is enabled by replacing the ionisable group of cysteine with another species with suitable ionisation properties to permit isoelectric focussing to be performed on the derivatised proteins. This is critical, since the negative charge of cysteine is important in counteracting the positive charges of basic amino acids at basic pH values. Removal of the negative charge of cysteine causes the isoelectric point of many proteins to become much more basic, often moving them outside the range conveniently analysed by isoelectric focussing.

The inventive method can be applied prior to the isoelectric focussing step of two dimensional electrophoresis, or prior to the use of isoelectric focussing (IEF) which is followed by any subsequent analytical process.

By "peptides and/or proteins" is meant peptides and/or proteins of any size and shape, as well as modified peptides and/or proteins like glycoproteins or lipoproteins. Also meant are molecules which contain modified amino acids which are connected by one or more peptide bonds, such as proteins where e.g. some or all lysines and/or some or all of one or more other types of amino acid side chain have been alkylated or modified in some way so as to no longer resemble biological amino acid side chains.

The molecules suitable for the methods disclosed here alkylate cysteine with the same chemistry as iodoacetamide. Such molecules could be carboxyesterised iodoacetyltyrosine itself, or a similar molecule such as β-(4-hydroxyphenyl)ethyl iodoacetamide (Pierce, USA), which is in result iodoacetyltyrosine without the carboxyl group. Obviously the ethyl group of β-(4-hydroxyphenyl)ethyl iodoacetamide could be any alkyl group or derivative thereof, and all variants thereof are claimed. The alkyl group may contain any combination of atoms of any nature which do not alter the fundamental fact that the pKₐ of the hydroxyphenyl group approximates the pKₐ of cysteine, and thereby enables cysteine alkylation prior to isoelectric focussing to produce a 2D-PAGE pattern similar to that of proteins with underivatised cysteine residues. The pKₐ of the hydroxyphenyl group approximates that of cysteine, and thus use of this class of reagents for the alkylation of cysteine permits pre-isoelectric focussing labelling of proteins with iodine.

Furthermore the alkylating reagent could be any hydroxyphenylalkyl iodoacetamide, or any hydroxyaromatic alkyl iodoacetamide, wherein the alkyl group may contain any combination of atoms of any nature which do not alter the fundamental fact that the pKₐ of the hydroxyaromatic group approximates the pKₐ of cysteine. Further the alkylating reagent is any iodoacetamylaminoalkylphenyl-sulfonic acid, or any iodoacetamylaminoalkylphenylalkyl-sulfonic acid, or any iodoacetamylaminoalkylphenylcarboxylic acid, or any iodoacetamylaminoalkylphenylalkylcarboxylic acid, wherein the alkyl group may contain any combination of atoms of any nature (showing the above fundamental fact), and any phenyl group may contain additional such alkyl groups.

One further alkylating reagent to be isotopically labelled prior to isoelectric focussing is THIOLYTE® Monobromobimane (Calbiochem, USA), which exhibits almost the identical pKₐ of cysteine. Another alkylating reagent to be isotopically labelled is THIOLYT® p-Sulfobenzoyloxybromobimane (Calbiochem, USA) or THIOLYTE® Monobromotrimethylammoniobimane (Calbiochem, USA), which exhibit different pKₐ-values to cysteine.

Another class of molecules disclosed here for radioactive labelling of peptides or proteins on cysteines include the Hudson-Weber Reagent N-(iodoacetylaminoethyl)-5-napthylamine-1-sulfonic acid (1,5-I-AEDANS) and its 1,8 isomer (6). In these molecules there are two ionisable groups, a sulfonic acid and a secondary amine. The sulfonic acid has a pKₐ below the range of the normal working pH range for isoelectric focussing. The secondary amine has partial aromatic character, and thus has a pKₐ much lower than the 9.5-10.5 which would be expected for a secondary amino group. When cysteine is derivatised with these molecules the resulting modified amino acid side chain is neutrally charged below the pKₐ of the secondary amine, and negatively charged above that pKₐ, which mimics the ionisation of cysteine in progressing from neutral to negative charge with increasing pH, albeit at a lower pKₐ for 1,5-I-AEDANS. This class of molecules is useful to alter the isoelectric point (pI) of cysteine-containing proteins such that they adopt a more acidic pH.

This alkylation results in a distinctly different 2D-PAGE pattern than that observed for proteins which contain native cysteine. However this is advantageous in that many proteins from more basic regions of the gel, where IEF is difficult, can be visualised in more acidic regions of the isoelectric focussing gel.

Also comprised in the above-mentioned class of molecules are all N-iodoacetylaminoalkyl versions of N-(iodoacetylaminoethyl)-5-napthylamine-1-sulfonic acid (1,5-I-AEDANS) and its 1,8 isomer. By "alkyl" is meant also groups which contain any other combination of atoms. This includes large electron rich groups which affect the pKₐ of the acidic or of the basic groups of the AEDANS core structure. Also comprised by the invention are molecules which introduce another ionisable moiety in the alkyl group, and thus behave recognisably different during isoelectric focussing. All molecules where a further non-ionisable chemical group is added to the napthyl ring structure of such above disclosed molecules are also comprised. This includes additional aromatic elements.

Additionally, all molecules where a carboxylic acid group or a phosphoric acid group replaces the sulfonic acid group are included, as are all molecules where the napthyl ring structure is replaced by a phenolic ring, and all combinations of the above variations. Therefore the use of a zwitterionic N-iodoacetylaminoalkyl molecule containing a secondary, tertiary or quarternary amine base and an acidic group (sulfonic acid, carboxylic acid, phosphoric acid) on an aromatic ring structure, which could be iodinated, are claimed for the radioactive labelling of cysteine residues with iodine.

In a further preferred embodiment of the inventive method excess alkylating reagent is reacted with an excess of another molecule to impart a charge to the unreacted alkylating molecule, whereby the excess alkylating reagent is very efficiently removed from the experimental system during electrophoresis. Hence, these molecules greatly improve the specific signal to noise ratio of the sample. The type of molecules disclosed for this purpose include thioglycolic acid and its salts, glutathione, 3-mercapto-1-propanesulfonic acid and its salts, and cysteamine. These molecules all have free sulfhydryl groups which attack the activated carbon of iodoacetamide-like reagents, forming a thioether linkage, as well as having an additional ionisable group which affects the charge of the unreacted alkylating molecule: which are the distinguishing features of the molecules disclosed for this purpose. Cysteamine adds a group with pKₐ of approximately 10.4, and is most suitable for electrophoretically removing alkylating reagents from less basic pH gradients, such as pH 3-8 or 4-9. However with appropriate control of electrophoresis conditions, alkylating reagents which have been thioetherised with cysteamine can also be induced to migrate out of more basic pH gradients, since the cysteamine amine group remains partially charged even at pH 12 and above. Thioglycolate, glutathione, and 3-mercapto-1-propane-sulfonate add a negative charge to the alkylating reagent, and are therefore most suitable for removing unreacted alkylating reagent from more basic pH gradients. The pKₐ of the thioglycolate carboxyl group is somewhat higher than that of the aspartate and glutamate side chains, however with appropriate control of electrophoresis conditions such molecules can also be induced to migrate out of quite acidic pH gradients. Thioglycolate imposes less disruption of the pH gradient in acidic regions during isoelectric focussing at similar concentrations to 3-mercapto-1-propanesulfonate, and therefore is in many cases preferable to 3-mercapto-1-propanesulfonate. Glutathione introduces two carboxylic acid groups to the alkylating molecule, with pKₐ values approximating those of the aspartate and glutamate side chains, and is in most cases the reagent of choice. The use of thioglycolate in the presence of nonalkylated cysteine results in a low level of nucleophilic attack of the cysteine sulfur on the beta carbon of thioglycolate, with the sulfhydryl of thioglycolate acting as leaving group in a similar chemistry to iodoacetamide attack by cysteine. Therefore this reagent is most useful in applying after quantitative cysteine alkylation in 2D-PAGE and other proteomic studies.

Also in the case of fluorescent compounds as labels, unreacted fluorescent compounds can be removed by reaction with e.g. thioglycolate, glutathione, mercapto-1-propansulfonate, or cysteamine. I.e. this method of background signal removal is not restricted to the use of radioactive signals.

One preferred label in connection with the inventive method is iodine. Nevertheless, there are other examples of appropriate labels. These include radioactive isotopes of carbon, hydrogen, sulfur, selenium (e.g. in the position of the sulfonic acid group with sulfur substitute for selenium), or phosphate, which can be incorporated into peptides or proteins by proper use of the corresponding above classes of molecules. The peptides or proteins alkylated by all the above methods finally containing these radioactive isotopes can be subsequently quantified and/or analysed by means of isotopic detection such as autoradiography, phosphorimager, or other methods. Furthermore the use of radioactively excited fluorescing compounds is included.

In a preferred embodiment of the invention the cysteine alkylating reagent is derivatized with radioactive isotopes, which exhibit coincident emission upon decay and are compatible with multi photon detection (MPD).

In another embodiment labelling is performed with different isotopes, which permits the identification of differentially labelled proteins by virtue of differing masses of modified cysteine side chains.

In one preferred embodiment of the inventive method there are specific and distinguishable isotopes incorporated into proteins from different sources, so that identical proteins from different sources can be co-electrophoresed in the same gel but quantified separately by virtue of their resultant differential molecular masses. In this embodiment peptides generated from proteins from different sources have identical masses until a cysteine was reached in the primary amino acid sequence. At this point the mass of peptides from both species differs by at least one atomic unit, which is easily detectable by e.g. modern mass spectrometers. Thus a differential display using a "multi colour" readout system is enabled. Importantly, this methodology does not require radioactive labelling of the proteins. The isotopes can be isotopes of any of the atoms from which the alkylating reagent or its reaction products are derived, e.g. carbon, hydrogen, nitrogen, sulfur, oxygen, iodine, phosphate, or selenium. This embodiment of the invention is e.g. applicable to proteins harvested from small amounts of primary human biopsies from the clinics, or to organisms or samples collected in the field, such as for food quality control testing, or assaying for the presence of specific micro-organisms.

The alkylating reagent can be labelled with specific isotopes before or after the reaction of the alkylating reagent with the proteins. Nevertheless, labelling of the alkylating reagent prior to alkylation is preferred because labelling the molecules after alkylation could produce non-specific labelling of other residues which could interfere with identification by e.g. mass analysis.

One embodiment of the inventive method is disclosed as follows. However it is recognised that others are also possible, and the claims are not intended to be restricted to this embodiment only. Proteins are solubilised by boiling in an SDS-containing buffer with reducing agent, e.g. β-mercaptoethanol (β-ME), dithiothreitol (DTT), tributylphosphine (TBP), or others, according to Laemmli (7). Under these buffer conditions the molecule is alkylated in the presence of 1-5 mM iodoalkylating reagent (e.g. carboxyesterised iodoacetyltyrosine or β-(4-hydroxyphenyl)ethyl iodoacetamide). The reducing environment is then removed by dilution or dialysis into another buffer, by direct oxidation, or by precipitation of analyte molecules and resuspension in another buffer. Because of the alkylation of cysteines there is no longer a requirement for reducing conditions to prevent disulfide formation. In the subsequent buffer the iodination of tyrosine and modified cysteine amino acid side chains is performed according to standard chemistry. The resulting reaction mixture containing iodinated proteins can be then either directly applied to isoelectric focussing after dilution into a suitable buffer, or proteins can be precipitated and resuspended into a suitable buffer.

In one embodiment of the invention non-radioactively labelled proteins present in the diluting buffer are used in order to minimise loss of radioactive proteins from the analysis due to non-specific binding to experimental surfaces or matrices in very dilute solutions of radioactive protein.

For solubilisation of proteins as mentioned above there are several general possibilities. For example the proteins are solubilised in an aqueous buffer, in an organic buffer, in a urea-based or a urea-thiourea-based buffer, in a buffer containing cationic detergents or surfactants, especially SDS, in a buffer containing anionic detergents or surfactants, in a buffer containing nonionic detergents or surfactants, in a buffer containing amphoteric or zwitterionic detergents or surfactants, in a buffer containing polyionic detergents or surfactants, or in a buffer containing a mixture of the above detergents or surfactants.

Said buffer for solubilisation could contain one or more sulfhydryl reducing agents (β-ME, DTT, etc), e.g. one or more phosphine reducing agents (e.g. TBP), or other reducing agents, or the buffer could contain a mixture of reducing agents. It has to be considered that the presence of sulfhydryl reducing agents during alkylation reduces the efficiency of protein alkylation by competition with the sulfhydryl group of cysteine. Therefore it is preferred to use phosphines or other non-sulfhydryl reducing agents to generate the reducing environment before alkylation.

The reaction volume during alkylation is preferably below or equal to 25 microlitres. Nevertheless it could also be above 25 microlitres.

The inventive method is especially applicable in the field of protein analysis, e.g. in the pre- or post-isoelectric derivatisation of cysteines for two dimensional electrophoresis. It is also useful in the pre- or post-electrophoretic derivatisation of cysteines for free flow electrophoresis or for capillary electrophoresis, or for other methods conventionally used for protein analysis. By way of example, proteins may be separated and analysed on the basis of isoelectric point (e.g. by chromatofocussing or isoelectric focussing), of electrophoretic mobility (e.g. by non-denaturing electrophoresis or by electrophoresis in the presence of a denaturing agent), by chromatography, including FPLC and HPLC, on any suitable matrix (e.g. gel filtration chromatography, ion exchange chromatography, reverse phase chromatography or affinity chromatography) or by centrifugation.

The inventive method could also be used in the pre- or post-electrophoretic, or pre- or post-chromatographic separation derivatisation of cysteines on proteins analysed by electrophoresis or chromatography on a micro chip, or any process which an expert in the art would recognise as similar in principle of operation to prior art techniques in this field. By "micro chip" is meant a micro-apparatus of the type where electrical circuitry or microfluidic channels can be created by modern methods such as photoetching. Included in this definition are micro chips which possess regions of differing chromatographic properties to differentially bind proteins to discrete subregions of the micro chip. This includes micro chips which present arrays of specific nucleic acids, such as aptamers, or specific proteins, such as antibodies or bacteriophage coat proteins.

Furthermore the inventive method is applicable in the pre- or post-electrophoretic or -chromatographic, or pre- or post-separation derivatisation of cysteines on proteins analysed after microfractionation into distinct receptacles or chambers following chromatography, electrophoresis, or other separation processes. By "receptacles or chambers" is meant vessels of containment, such as miniature reaction test tubes, and also micro-arrays etched into a surface of glass or other material, or into a micro chip as described above.

As an example of the performing of the inventive method the peptides and/or proteins of a sample are solubilised by boiling in an SDS-containing buffer with reducing agent. Then, alkylation of amino acid residues, especially of cysteine residues, of the peptides and/or proteins is performed by incubating the sample with β-(4-hydroxyphenyl)ethyl iodoacetamide, wherein the isoelectric point of the peptides and/or proteins is essentially the same before and after alkylation. Iodination of tyrosine and alkylated cysteine amino acid side chains of the peptides and/or proteins is performed by standard chemistry. In another embodiment the alkylating reagent is labelled prior to alkylation of the peptides and/or proteins. Then the alkylating reagent contains e.g. one or more radioactive atoms. Following alkylation of the peptides and/or proteins excess alkylating reagent is reacted with e.g. glutathione to impart a net charge to the alkylating reagent permitting removal of excess alkylating reagent during electrophoresis. Low molecular weight molecules could be removed by dialysis prior applying the labelled sample to isoelectric focussing.

### REFERENCES

1. Andrews, A. T. 1986. Electrophoresis : Theory, Techniques, and Biochemical and Clinical Applications, 2nd Ed. (Dec 1986) ed. Oxford University Press. ISBN 0198546327.
2. Creighton, T. E. 1984. Proteins: Structures and molecular properties. Freeman and Company, New York. ISBN .
3. Drukier, A. K. Oct. 22 1997. Improved quantitation of gamma and X-ray emitting isotopes. European patent EP1994000930101.
4. Drukier, A. K. July 2 1996. Quantitation of gamma and X-ray emitting isotopes. USA patent 5532122.
5. Drukier, A. K., and I. R. Sagdejev. Sept. 30 1998. Ultralow background multiple photon detector. European patent EP1996000941945.
6. Hudson, E. N., and G. Weber. 1973. Synthesis and characterization of two fluorescent sulfhydryl reagents. Biochemistry 12:4154-61.
7. Laemmli, U. K. 1970. Cleavage of structural proteins during the assembly of the head of bacteriophage T4. Nature 227:680-5.
8. Righetti, P. G. (eds.). 1990. Immobilised pH gradients: theory and methodology, vol. 20. Elsevier, Amsterdam.
9. Righetti, P. G., A. Bossi, E. Wenisch, and G. Orsini. 1997. Protein purification in multicompartment electrolyzers with isoelectric membranes. J Chromatogr B Biomed Sci Appl 699:105-15.
10. Wasinger, V. C., S. J. Cordwell, A. Cerpa-Poljak, J. X. Yan, A. A. Gooley, M. R. Wilkins, M. W. Duncan, R. Harris, K. L. Williams, and I. Humphery-Smith. 1995. Progress with gene-product mapping of the Mollicutes: Mycoplasma genitalium. Electrophoresis 16:1090-4.
11. Weber, G., and P. Bocek. 1998. Stability of continuous flow electrophoresis [In Process Citation]. Electrophoresis 19:3094-5.
12. Westermeier, R. 1977. Electrophoresis in Practice : A Guide to Methods and Applications of DNA and Protein Separations, 2n ed. John Wiley and Sons, Weinhem. ISBN 3527300708.
13. Wilkins, M. R., K. L. Williams, R. D. Appel, and D. F. Hochstrasser (eds.). 1997. Proteome Research: New Frontiers in Functional Genomics. Springer, Berlin.

## Claims

1. Method for isotopically labelling peptides and/or proteins in preparative and analytical electrophoresis, especially as sample preparation for isoelectric focussing, wherein side chains of specific amino acid residues of said peptides and/or proteins are derivatized and at least one label is incorporated into the derivatized side chains of peptides and/or proteins.

2. Method according to claim 1, characterized in that the isoelectric point of said peptides and/or proteins is essentially the same before and after derivatisation.

3. Method according to claim 1, characterized in that the isoelectric point of said peptides and/or proteins is altered by the derivatisation.

4. Method according to one of claims 1 to 3, characterized in that said side chains contain sulfhydryl groups, wherein preferably said amino acid residues are those of cysteines.

5. Method according to one of the preceding claims, characterized in that said side chains are derivatized by alkylation with an alkylating reagent, wherein preferably the alkylating reagent already comprises the label to be incorporated into the proteins and/or peptides.

6. Method according to claim 5, characterized in that said alkylating reagent is carboxyesterised iodoacetyltyrosine.

7. Method according to claim 5, characterized in that said alkylating reagent is a hydroxyphenylalkyl iodoacetamide, especially β-(4-hydroxyphenyl)ethyl iodoacetamide, or a hydroxyaromatic alkyl iodoacetamide.

8. Method according to claim 5, characterized in that said alkylating reagent is iodoacetamylaminoalkylphenyl-sulfonic acid or iodoacetamylaminoalkylphenylalkyl-sulfonic acid or iodoacetamylaminoalkylphenyl-carboxylic acid or iodoacetamylaminoalkylphenylalkyl-carboxylic acid.

9. Method according to claim 5, characterized in that said alkylating reagent is monobromobimane.

10. Method according to claim 5, characterized in that said alkylating reagent is p-sulfobenzoyloxybromobimane or monobromotrimethylammoniobimane.

11. Method according to claim 5, characterized in that said alkylating reagent is a N-(iodoacetylaminoalky)-5-napthylamine-1-sulfonic acid or a N-(iodoacetylaminoalkyl)-8-napthylamine-1-sulfonic acid, especially N-(iodoacetylaminoethyl)-5-napthylamine-1-sulfonic acid (1,5-I-AEDANS) or N-(iodoacetylaminoethyl)-8-napthylamine-1-sulfonic acid.

12. Method according to claim 11, characterized in that the sulfonic acid group of said alkylating reagent is replaced by a carboxylic acid group and/or a phosphoric acid group and/or the napthyl ring structure of the reagent is replaced by a phenolic ring.

13. Method according to one of claims 5 to 12, characterized in that excess alkylating reagent is reacted with an excess of another molecule, expecially thioglycolic acid and/or its salts, 3-mercapto-1-propane-sulfonic acid and/or its salts, glutathione, and/or cysteamine, thereby imparting a charge to the excess alkylating reagent.

14. Method according to one of the preceding claims, characterized in that the label is a radioactive isotope, especially a radioactive isotope out of the group comprising carbon, hydrogen, sulfur, selenium, phosphate and iodine, wherein iodine is preferred.

15. Method according to claim 14, characterized in that said radioactive isotope exhibits coincident X-rays and gamma ray emission upon decay and is compatible with muliple photon detection (MPD).

16. Method according to one of the preceding claims, characterized in that different isotopes of the same element are incorporated as labels, especially different isotopes of iodine, permitting co-electrophoresis of different samples and/or individual identification of differentially labelled peptides and/or proteins by virtue of differing radioactive decay and/or differing masses, especially by mass spectrometry.
